# EUROPEAN PATENT APPLICATION

(11) **EP 2 692 352 A1**
(43) Date of publication of application: **05.02.2014**
(21) Application number: 12765928.2
(22) Date of filing: 30.03.2012
(51) Int. Cl.: A61K 38/00, A61K 45/00, A61P 15/12, C12Q 1/02, C12Q 1/68, G01N 33/15, G01N 33/50, C07K 14/47, C12N 15/09

(54) **HOT FLASH SUPPRESSANT**

(30) Priority: 31.03.2011 JP 2011080428
(71) Applicant: Shiseido Company, Ltd., Tokyo 104-0061 (JP)
(72) Inventor: SAWANE, Mika, Yokohama-shi Kanagawa 236-8643 (JP); KAJIYA, Kentaro, Yokohama-shi Kanagawa 236-8643 (JP); TAKAGI, Masaya, Yokohama-shi Kanagawa 236-8643 (JP)
(74) Representative: O'Gara, Margaret Mary
(86) International application number: PCT/JP2012/058687
(87) International publication number: WO 2012/133825

(57) **Abstract**

The present invention provides: a hot flash suppressant comprising apelin or an apelin receptor agonist, wherein the apelin receptor agonist is selected from the group consisting of Rubus buergeri, Aster yomena, Stauntonia hexaphylla, Rubus grayana, Kalimeris indica, Lithocarpus edulis, Myrica rubra, Syzgium jambos, Quercus serrata and extracts of these plants, as well as a saffron extract, a pine extract, a cinchona extract, a comfrey extract, a Scutellaria extract, a rosemary extract, an apricot seed powder, a gentian extract, a thyme extract, a mint powder, a jujuba fruit extract, a hop extract, a kiwifruit extract, a roman chamomile extract, an apple extract, a hawthorn extract and a turmeric extract; and a screening method for a medical agent for improving hot flash.

SEQUENCE LISTING

<110>  Shiseido Co., Ltd.

<120>  Hot Flash Inhibitory Agent

<130>  AA551-PCT

<160>  2

<170>  PatentIn version 3.3

<210>  1
<211>  36
<212>  PRT
<213>  Homo sapiens

<400>  1

Leu Val Gln Pro Arg Gly Ser Arg Asn Gly Pro Gly Pro Trp Gln Gly
1               5                   10                  15

Gly Arg Arg Lys Phe Arg Arg Gln Arg Pro Arg Leu Ser His Lys Gly
            20                  25                  30

Pro Met Pro Phe
        35

<210>  2
<211>  108
<212>  DNA
<213>  Homo sapiens

<400>  2
ctggtgcagc ccagagggtc aaggaatggg ccagggccct ggcagggagg tcggaggaaa      60

ttccgccgcc agcggccccg cctctcccat aagggaccca tgcctttc                  108

## Description

### TECHNICAL FIELD

The present invention relates to a suppressant of hot flashes caused by menopausal disorders and to a method for improving hot flashes.

### BACKGROUND ART

Hot flashes (characterized by a localized rise in body surface temperature and accompanying flushing, warmth, redness and excessive perspiration) constitute a menopausal disorder that is a typical symptom of the climacteric / postmenopausal syndrome. A decrease in female hormones (such as estrogen) is thought to cause menopausal disorders including hot flashes, and young women may also present with symptoms similar to menopausal disorders brought on by disruption of hormonal balance caused by excessive stress or dietary factors and the like. Female hormone replacement therapy may be performed as a method for treating these menopausal disorders and symptoms similar thereto. However, since female hormone replacement therapy is known to cause side effects in the form of increased risk of breast cancer, endometrial cancer and other forms of cancer, long-term female hormone replacement therapy is not desirable. Compounds serving as antagonists of CGRP, which has vasodilatory properties (Japanese Unexamined Patent Publication (Kokai) No. 2008-525510) and antagonists of gonadotropin releasing hormone, which is also involved ir hot flashes (Published Japanese Translation of PCT International Publication for Patent Application (Kohyo) No. 2002-512976, Japanese Unexamined Patent Publication (Kokai) No. 2008-195728), have been previously identified for the prevention or treatment of hot flashes as alternatives to female hormone replacement therapy.

Apelin is a molecule that was isolated in 1998 from cell extracts of the bovine stomach as a binding factor to APJ, a seven-pass transmembrane, G protein-coupled receptor that was long considered to be an orphan receptor. Although apelin cDNA encodes 77 amino acids, a long form (42 to 77 amino acids) and a short form (65 to 77 amino acids) are formed from its precursor. Both of these forms of apelin are known to induce activation of APJ. APJ has previously been reported to be expressed in the cardiovascular system and central nervous system, and is suggested to be involved in mechanisms for regulating body fluids, such as by demonstrating a myocardial contractile action in the heart or by controlling the expression of vasopressin in the nervous system. In addition, since APJ is also involved in infections by functioning as a receptor for the AIDS virus, it has suddenly attracted attention as a target of drug development from various viewpoints. In the vascular system, APJ is thought to be expressed in vascular endothelial cells and parietal cells, and since gene knockdown experiments using Xenopus laevis have indicated that the apelin/APJ system has an essential role in vascular development, and the expression of this receptor has been observed in endothelial cells of mice and humans, it has been predicted to be involved in vascularization in mammals (Experimental Medicine, Vol. 26, No. 9(2008), pp. 1380-1383). In addition, the apelin secreted when vascular endothelial cells are stimulated by Ang1 has been reported to control caliber size through analyses of apelin knockout mice and in vitro vascular analyses (Kidoya, et al. (2008) Spatial and Temporal Role of the Apelin/APJ System in the Caliber Size Regulation of Blood Vessels during Angiogenesis, EMBO J. 27:522-534). However, the involvement of apelin in the suppression and improvement of hot flashes has previously been completely unclear.

### DISCLOSURE OF THE INVENTION

### Problems to be Solved by the Invention

Pharmaceuticals that demonstrate adequate clinical effects with respect to suppression or improvement of hot flashes in one form of menopausal disorder have not been reported. Therefore, an object of the present invention is to provide a hot flash suppressant and a method for improving hot flashes. Furthermore, in the present invention, a suppressant refers to that which includes therapeutic agents, preventive agents and ameliorants, while a method for improving refers to that which includes a therapeutic method, preventive method or suppression method.

### Means for Solving the Problems

In order to investigate the relationship between apelin and local rises in body surface temperature attributable to menopausal disorders along with the accompanying warmth, the inventors of the present invention compared tail temperatures following administration of a vasodilator in mice that had undergone ovariectomy and been inserted with a gene that causes apelin to be highly expressed in the epidermis (menopausal mice highly expressing apelin) and wild type mice that had undergone ovariectomy (menopausal wild type mice). Measurement of changes in tail temperature following administration of a vasodilator to menopausal wild type mice has been established as a model of menopausal hot flashes (Kai, M., Yamauchi, A., Tominaga, K., Koga, A., Kai, H., Kataoka, Y.: Soybean isoflavones eliminate nifedipine-induced flushing of tail skin in ovariectomized mice, J. Pharmacol. Sci. 2004, 95: 476-478). In this experiment, the inventors of the present invention confirmed that, although elevated skin temperature persisted for a long period of time in the menopausal wild type mice, tail temperature fell to the normal value in a comparatively short period of time in the menopausal mice highly expressing apelin in comparison with the menopausal wild type mice. This result indicates that apelin contributes to suppression or improvement of hot flashes.

The inventors of the present invention also provide a method of screening hot flash suppressants based on the aforementioned findings. This method is characterized by selecting a pharmaceutical agent that increases expression of intrinsic apelin in skin cells, or a pharmaceutical agent that activates apelin receptors, for use as a hot flash suppressant. Increased expression of intrinsic apelin can be determined by, for example, directly measuring the amount of apelin in skin cells, while activation of apelin receptors can be determined by measuring calcium ion concentration or cAMP concentration and the like in skin cells. Pharmaceutical agents that activate apelin receptors as determined in this manner, namely apelin receptor agonists, are preferably substances that have an apelin-like action.

When the inventors of the present invention developed a screening method utilizing the apelin/APJ signal system and used that system to screen various substances, they surprisingly found that *Rubus buergeri, Aster yomena, Stauntonia hexaphylla, Rubus grayana, Kalimeris indica, Lithocarpus edulis, Myrica rubra, Syzygium jambos, Quercus serrata* and extracts of these plants, as well as saffron extract, pine extract, cinchona extract, comfrey extract, Scutellaria extract, rosemary extract, apricot seed powder, gentian extract, thyme extract, mint powder, jujuba fruit extract, hop extract, kiwifruit extract, roman chamomile extract, apple extract, hawthorn extract and turmeric extract, activate APJ and have a similar function to that of apelin. Thus, these plants are useful for the suppression, improvement, treatment and/or prevention of hot flashes in the same manner as apelin.

The present application includes the inventions indicated below.
(1) A hot flash suppressant comprising apelin or an apelin receptor agonist.
(2) The hot flash suppressant of (1), wherein
   the apelin is:
   (a) a protein composed of the amino acid sequence represented by SEQ ID NO: 1, or
   (b) a protein composed of an amino acid sequence, in which one or several amino acids in the amino acid sequence of SEQ ID NO: 1 have been deleted, substituted or added, and has apelin activity.
(3) The hot flash suppressant described in (1), wherein the apelin receptor agonist is selected from the group consisting of *Rubus buergeri, Aster yomena, Stauntonia hexaphylla, Rubus grayana, Kalimeris indica, Lithocarpus edulis, Myrica rubra, Syzygium jambos, Quercus serrata* and extracts of these plants, as well as saffron extract, pine extract, cinchona extract, comfrey extract, Scutellaria extract, rosemary extract, apricot seed powder, gentian extract, thyme extract, mint powder, jujuba fruit extract, hop extract, kiwifruit extract, roman chamomile extract, apple extract, hawthorn extract and turmeric extract.
(4) A method for treating, improving or preventing hot flashes, comprising administration of apelin or an apelin receptor agonist to a subject requiring improvement of hot flashes.
(5) The method for treating, improving or preventing hot flashes described in (4), wherein the apelin is:
   (a) a protein composed of the amino acid sequence represented by SEQ ID NO: 1, or
   (b) a protein composed of an amino acid sequence, in which one or several amino acids in the amino acid sequence of SEQ ID NO: 1 have been deleted, substituted or added, and has apelin activity.
(6) The method for treating, improving or preventing hot flashes described in (4), wherein the apelin receptor agonist is selected from the group consisting of *Rubus buergeri, Aster yomena, Stauntonia hexaphylla, Rubus grayana, Kalimeris indica, Lithocarpus edulis, Myrica rubra, Syzygium jambos, Quercus serrata* and extracts of these plants, as well as saffron extract, pine extract, cinchona extract, comfrey extract, Scutellaria extract, rosemary extract, apricot seed powder, gentian extract, thyme extract, mint powder, jujuba fruit extract, hop extract, kiwifruit extract, roman chamomile extract, apple extract, hawthorn extract and turmeric extract.
(7) A use of apelin or an apelin receptor agonist for suppressing hot flashes.
(8) The use for suppressing hot flashes described in (7), wherein the apelin is:
   (a) a protein composed of the amino acid sequence represented by SEQ ID NO: 1, or
   (b) a protein composed of an amino acid sequence, in which one or several amino acids in the amino acid sequence of SEQ ID NO: 1 have been deleted, substituted or added, and has apelin activity.
(9) The use for suppressing hot flashes described in (7), wherein the apelin receptor agonist is selected from the group consisting of *Rubus buergeri, Aster yomena, Stauntonia hexaphylla, Rubus grayana, Kalimeris indica, Lithocarpus edulis, Myrica rubra, Syzygium jambos, Quercus serrata* and extracts of these plants, as well as saffron extract, pine extract, cinchona extract, comfrey extract, Scutellaria extract, rosemary extract, apricot seed powder, gentian extract, thyme extract, mint powder, jujuba fruit extract, hop extract, kiwifruit extract, roman chamomile extract, apple extract, hawthorn extract and turmeric extract.
(10) A use of apelin or an apelin receptor agonist for producing a preparation for suppressing hot flashes.
(11) The use of (7), wherein the apelin is:
   (a) a protein composed of the amino acid sequence represented by SEQ ID NO: 1, or
   (b) a protein composed of an amino acid sequence, in which one or several amino acids in the amino acid sequence of SEQ ID NO: 1 have been deleted, substituted or added, and has apelin activity.
(12) The use described in (7), wherein the apelin receptor agonist is selected from the group consisting of *Rubus buergeri, Aster yomena, Stauntonia hexaphylla, Rubus grayana, Kalimeris indica, Lithocarpus edulis, Myrica rubra, Syzygium jambos, Quercus serrata* and extracts of these plants, as well as saffron extract, pine extract, cinchona extract, comfrey extract, Scutellaria extract, rosemary extract, apricot seed powder, gentian extract, thyme extract, mint powder, jujuba fruit extract, hop extract, kiwifruit extract, roman chamomile extract, apple extract, hawthorn extract and turmeric extract.
(13) An apelin receptor agonist selected from the group consisting of *Rubus buergeri, Aster yomena, Stauntonia hexaphylla, Rubus grayana, Kalimeris indica, Lithocarpus edulis, Myrica rubra, Syzygium jambos, Quercus serrata* and extracts of these plants, as well as saffron extract, pine extract, cinchona extract, comfrey extract, Scutellaria extract, rosemary extract, apricot seed powder, gentian extract, thyme extract, mint powder, jujuba fruit extract, hop extract, kiwifruit extract, roman chamomile extract, apple extract, hawthorn extract and turmeric extract.
(14) A method for screening hot flash suppressants by using as an indicator thereof apelin receptor activity or apelin expression.
(15) A hot flash suppressant obtained according to the screening method described in (14).
(16) A hot flash suppressant containing a vector containing a gene that encodes apelin.

### Effects of the Invention

Use of the hot flash suppressant of the present invention containing apelin or an apelin receptor agonist makes it possible to suppress, treat or prevent the menopausal disorder of hot flashes, namely a local rise in body surface temperature, as well as its accompanying symptoms such as warmth, flushing, redness or excessive perspiration.

### BRIEF DESCRIPTION OF THE DRAWINGS

FIG. 1 indicates changes in tail temperature in menopausal wild type mice (OVXWT), menopausal mice highly expressing apelin (OVXTG) and wild type mice that underwent a sham surgery (shamWT) following administration of a vasodilator (nifedipine).

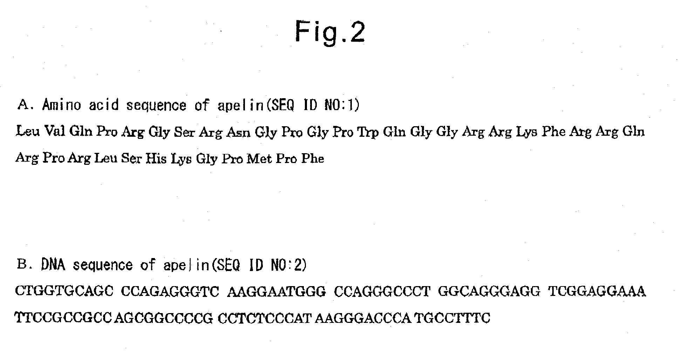

FIG. 2 shows the amino acid sequence (SEQ ID NO: 1) and DNA sequence (SEQ ID NO: 2) of human apelin.
FIG. 3 is a graph indicating a decrease in intracellular cAMP concentration induced by apelin and suppression of that decrease in intracellular cAMP concentration by apelin neutralizing antibody.

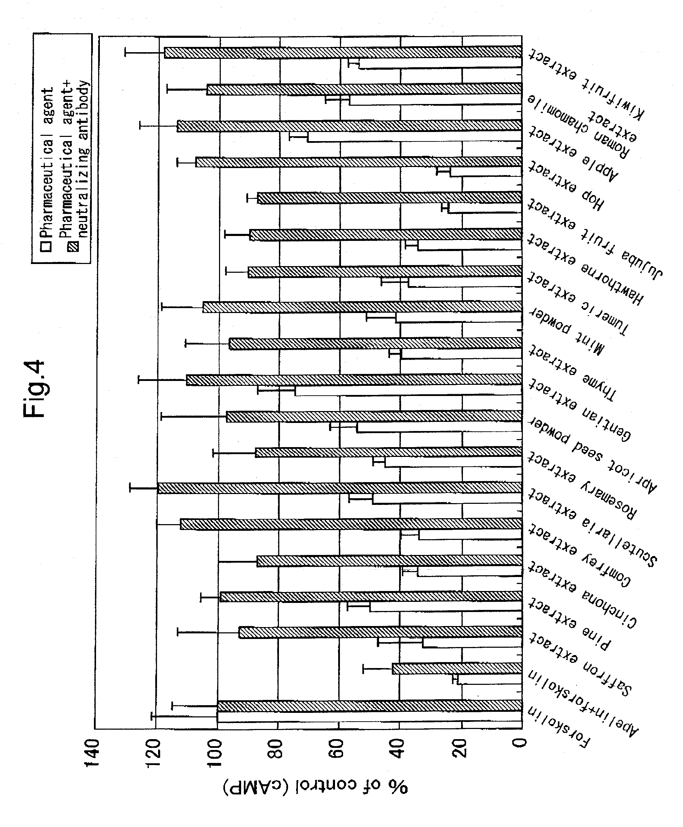

FIG. 4 is a graph indicating the ratios of increases in intracellular cAMP concentration versus a forskolin addition group serving as a control in the case of adding apelin-like pharmaceutical agents (saffron extract, pine extract, cinchona extract, comfrey extract, Scutellaria extract, rosemary extract, apricot seed powder, gentian extract, thyme extract, mint powder, jujuba fruit extract, hop extract, kiwifruit extract, roman chamomile extract, apple extract, hawthorn extract and turmeric extract), either alone (as indicated by the bars on the left in the graft) or in combination with apelin neutralizing antibody (as indicated by the bars on the right in the graph).
FIG. 5 is a graph indicating the ratios of increases in intracellular cAMP concentration versus a forskolin addition group serving as a control in the case of adding apelin-like pharmaceutical agents (extracts of *Rubus buergeri, Aster yomena, Stauntonia hexaphylla, Rubus grayana, Kalimeris indica, Lithocarpus edulis, Myrica rubra* and *Syzygium jambos*) either alone (as indicated by the bars on the left in the graft) or in combination with apelin neutralizing antibody (as indicated by the bars on the right in the graph).

### BEST MODE FOR CARRYING OUT THE INVENTION

One aspect of the present invention relates to a hot flash suppressant comprising apelin or an apelin receptor agonist.

Human apelin is reported to be expressed at various sites, such as the heart, lungs, kidneys, fat, stomach, brain, adrenals or skin, and is known to be a ligand of apelin APJ receptor composed of 36 amino acids derived from a precursor protein having 77 amino acids (Kawamata, Y., et al., (2001) Molecular Properties of Apelin: Tissue Distribution and Receptor Binding, Biochemica et Biophysica Acta., 2-3:162-171). In addition, mutants of apelin, in which amino acids on the C-terminal and/or N-terminal are missing, are known such as apelin-13 (polypeptide composed of the amino acid sequence from the 24th to 36th amino acids of apelin), and accordingly in the present invention, the term "apelin" includes not only full-length human apelin composed of the 36 amino acids represented by SEQ ID NO: 1, but also apelin mutants, and fragments thereof, in which one or several amino acids have been deleted, substituted or added, provided the activity of apelin is retained. The number of amino acids that are deleted, substituted or added is preferably 1 to 5, 1 to 3 or 1 to 2. In addition, the apelin includes not only human-derived apelin, but also that derived from rodents, such as rats or mice, or rabbits. Moreover, the apelin also includes apelin mutants composed of a sequence having sequence identity of at least 70%, preferably at least 80%, and more preferably at least 90%, with respect to full-length human apelin composed of the 36 amino acids represented by SEQ ID NO: 1. In a more preferable aspect, the aforementioned sequence identity of an apelin mutant is at least more than 95%, more preferably more then 98%, and even more preferably more than 99%.

Moreover, a gene that encodes apelin as referred to in the present description includes a gene that encodes apelin, a gene that encodes a polypeptide in which one or several nucleotides have been deleted, substituted or added and has an apelin activity, and a gene that encodes a polypeptide that hybridizes with a gene that encodes apelin under highly stringent conditions and has apelin activity. Here, "apelin activity" is defined as that which phosphorylates an intracellular signaling molecule in the form of Akt or reduces intracellular cAMP concentration as a result of binding to a receptor thereof in the form of APJ. Furthermore, highly stringent hybridization conditions refer to conditions comprising, for example, a sodium concentration of about 10 mM to 40 mM and preferably about 20 mM, and a temperature of about 50°C to 70°C and preferably about 60°C to 65°C.

APJ receptor is also referred to as angiotensin receptor-like 1 (AGTRL1), is a kind of G protein-coupled receptor (GPCR), and is composed of 380 amino acids. The transmembrane domain thereof exhibits homology of 40% to 50% with angiotensin (AT1) receptor. Apelin/APJ signals are downstream signals of ANG1/Tie2 in blood vessels, and have been reported to be involved in contraction of heart muscle, blood pressure, blood flow and vascularization (Kidoya, et al., (2008) Spatial and Temporal Role of the Apelin/APJ System in the Caliber Size Regulation of Blood Vessels during Angiogenesis, EMBO J. 27:522-534). APJ receptors are coupled with heterotrimeric G protein composed of an α subunit (about 40 kDa to 50 kDa), a β subunit (about 35 kDa) and a γ subunit (about 10 kDa), and are known to be related to an information transduction (signal) cascade. Although the α, β and γ subunits strongly bind when APJ receptors are not activated, when the APJ receptors are activated as a result of apelin binding thereto, an exchange reaction occurs between GDP and GTP bound to the α subunit, and they dissociate to a GTP-bound α subunit and β subunit. These subunits respectively activate target proteins or enzymes resulting in downstream signal transduction. Subsequently, GTP bound to the α subunit is decomposed by GTPase of the α subunit resulting in the formation of GDP, and the GDP-bound α subunit binds with the β and γ subunits to reform the inactive trimer. Trimeric G protein is classified into subfamilies such as Gs, Gi, Go, Gq, Gt or Golf based on functional and genetic differences in the α subunit. Gs and Gi respectively increase or suppress adenylate cyclase activity, Go is related to the signal transduction system of neural tissue, Gq activates phospholipase Cβ, and Gt and Golf respectively play important roles in the signal transduction systems of photoreceptor cells (retina) and olfactory cells. In the case APJ receptors have bound with apelin, the activity of adenylate cyclase is suppressed via the Gi pathway, thereby reducing intracellular cAMP concentration.

In the present invention, the term "apelin receptor agonist" is used in the same context as a substance that demonstrates the same action as apelin in the form of an apelin-like pharmaceutical agent, and refers to a substance that demonstrates the same effect as apelin through an apelin receptor. Although various substances are specified as apelin receptor agonists in the present description, they are not limited thereto.

Hot flashes in the present invention refer to symptoms caused by a disturbance in autonomic nerves controlled by the hypothalamus of the brain and a decrease in body temperature regulatory function accompanying a decrease in female hormones, and indicate symptoms such as a localized rise in body surface temperature and its accompanying warmth, flushing, redness and excessive perspiration. The hot flashes of the present invention refer to hot flashes that appear in the form of a menopausal disorder in particular.

In the present invention, a hot flash suppressant refers to a pharmaceutical agent that suppresses, alleviates, treats, improves or prevents the symptoms of hot flashes in the form of a localized rise in body surface temperature and its accompanying warmth, flushing, redness and excessive perspiration. Although the hot flash suppressant of the present invention contains apelin or an apelin receptor agonist, these components may be contained alone or a plurality of components may be used in combination. In the present invention, a suppressant refers to that which includes a therapeutic agent, preventive agent and ameliorant.

The hot flash suppressant of the present invention may be used alone or may be used in combination with a solvent, vehicle or other components, and the dosage, usage and drug form can be suitably determined according to the purpose of use thereof. For example, the dosage form of the suppressant of the present invention may be oral, parenteral or external. Examples of preparation forms include oral preparations such as tablets, powders, capsules, granules, extracts or syrups, parenteral preparations such as injection preparations, infusion preparations or suppositories, and external preparations such as ointments, creams, milky liquids, lotions or bath additives.

Although the incorporated amount of suppressant such as the apelin or apelin receptor agonist of the present invention in the hot flash suppressant of the present invention can be suitably determined according to the application, it is generally 0.00001% by weight to 20.0% by weight, and preferably 0.00001% by weight to 10.0% by weight, in the preparation.

In addition, the hot flash suppressant of the present invention can suitably incorporate, for example, a vehicle, desiccant, antiseptic, reinforcer, thickener, emulsifier, antioxidant, sweetener, acidifier, flavor enhancer, colorant or fragrance normally used in foods and pharmaceuticals, or a whitener, moisturizer, oily component, ultraviolet absorber, surfactant, thickener, alcohol, powdered component, dye, aqueous component, water or various skin nutritional agents normally used in cosmetics, as necessary in addition to the apelin or apelin receptor agonist.

In another mode of the present invention, the present invention relates to method for improving hot flashes comprising the administration of apelin or an apelin receptor agonist. In this case, the apelin or apelin receptor agonist is administered to a person suffering from symptoms of hot flashes in the form a localized rise in body surface temperature and any of its accompanying warmth, flushing, redness, excessive perspiration or combination thereof. The method for improving hot flashes of the present invention includes a therapeutic method, preventive method or suppression method. The apelin or apelin receptor agonist is administered by oral administration or parenteral administration (intravenously, intraarterially, transcutaneously or transmucosally).

In another mode of the present invention, the present invention relates to a method for screening hot flash suppressants that uses apelin receptor activity as an indicator thereof. This method can be carried out by using the apelin receptor activity of expression of apelin as an indicator.

The method for screening hot flash suppressants that uses apelin receptor activity as an indicator thereof is able to use apelin receptor activity as an indicator thereof by measuring calcium ion concentration or cAMP concentration in skin cells. Here, since a pharmaceutical agent that activates apelin receptors is an apelin receptor agonist, the present invention relates to a method for screening apelin receptor agonists. In the case a candidate pharmaceutical agent was able to lower calcium ion concentration or cAMP concentration in skin cells, that candidate pharmaceutical agent can be determined to be an apelin receptor agonist or hot flash suppressant. Moreover, in the case of carrying out secondary screening using an apelin receptor neutralizing antibody along with a pharmaceutical agent selected in this manner, and calcium ion concentration or cAMP concentration has recovered to a certain degree, that candidate pharmaceutical agent can be determined to be an apelin receptor agonist or hot flash suppressant. A pharmaceutical agent that activates apelin receptors, namely an apelin receptor agonist, which has been determined in this manner may be any arbitrary substance such as a compound or extract provided it activates apelin receptors, and is preferably an apelin-like peptide.

More specifically, the screening of apelin-like pharmaceutical agents of the present invention consists first of primary screening in which isolated cells (such as NIH-3T3 cells) transfected with a cAMP luciferase reporter vector are seeded into a 96-well plate, candidate pharmaceutical agents are added thereto and pre-incubated, and adenylate cyclase is activated with forskolin, followed by measuring the concentration of cAMP based on the luminescence of the luciferase as determined with a luminometer (such as the GloMax™ 96 Microplate Luminometer (Promega)), and selecting a pharmaceutical agent that has suppressed the increase in cAMP concentration by forskolin. Next, in order to confirm that the reduction in cAMP concentration is mediated by activation of APJ, secondary screening is carried out in which apelin neutralizing antibody (such as 4G5) is added to the pharmaceutical agent selected during primary screening, and a luciferase assay is carried out in the same manner as that during primary screening. As a result, a pharmaceutical agent for which the reduction in cAMP concentration is suppressed by addition of neutralizing antibody, and for which cAMP concentration recovers to 80% or more, preferably 90% or more, and most preferably 100%, can be selected as an apelin-like pharmaceutical agent.

A method for screening hot flash suppressants that uses intrinsic apelin as an indicator thereof is characterized by using the expression of intrinsic apelin in skin cells as an indicator, and selecting a pharmaceutical agent that increases expression of intrinsic apelin in skin cells as a hot flash suppressant. In one mode of the method for screening hot flash suppressants, the increase in expression of intrinsic apelin can be determined by directly measuring the amount of apelin in skin cells. Measurement of the amount of apelin in skin cells can be carried out by various methods using antibody specific for apelin that are well known in the relevant technical field, examples of which include immunostaining using a fluorescent substance, dye or enzyme, western blotting and immunoassay such as ELISA or RIA. In addition, the amount of apelin in skin cells can also be determined by extracting RNA from skin cells and measuring the amount of mRNA that encodes apelin. Extraction of mRNA and measurement of the amount thereof are well known in the relative technical field, and for example, quantification of RNA may be carried out by quantitative polymerase chain reaction (PCR). In addition to that described above, the expression level of apelin can also be determined by measuring a known biological activity of apelin. In addition, expression of apelin can also be determined by in situ hybridization or measurement of other biological activity. For example, if the expression of intrinsic apelin in skin cells has increased by 30% or more, preferably by 50% or more, more preferably by 70% or more, and most preferably by 100% in comparison with a control value, a candidate pharmaceutical agent may be judged to be a hot flash suppressant. Although there are no particular limitations on the control value, it may be, for example, the mean value of the expression level of intrinsic apelin in skin cells at a corresponding site in a statistically significant number of healthy individuals (such as 10 or more and preferably 100 or more).

In the present description, skin cells include epidermal cells, fibroblasts, vascular and lymphatic endothelial cells and adipocytes, epidermal cells in particular include keratinocytes, granular cells and prickle cells, and these cells may be of human origin or derived from other mammals such as rats, mice or rabbits.

In a preferable mode thereof, the aforementioned screening method further comprises confirmation of body temperature lowering effects at a site at which the effects of internal temperature can be eliminated by applying a candidate therapeutic agent having the ability to increase express of intrinsic apelin as previously described to an animal menopause model, such as an animal that has been ovariectomized, that has been administered a vasodilator. The site at which the effects of internal temperature can be eliminated is preferably the tail, and tail temperature can be measured using thermography.

The inventor of the present invention recently used a screening method that uses apelin receptor activity as an indicator thereof to obtain the findings that plants and extracts consisting of *Rubus buergeri, Aster yomena, Stauntonia hexaphylla, Rubus grayana, Kalimeris indica, Lithocarpus edulis, Myrica rubra, Syzygium jambos, Quercus serrata* and extracts of these plants, as well as saffron extract, pine extract, cinchona extract, comfrey extract, Scutellaria extract, rosemary extract, apricot seed powder, gentian extract, thyme extract, mint powder, jujuba fruit extract, hop extract, kiwifruit extract, roman chamomile extract, apple extract, hawthorn extract and turmeric extract, activate an apelin receptor in the form of APJ, and thereby have an extremely high likelihood of having functions similar to that of apelin. Thus, according to the present invention, an apelin receptor agonist is provided that comprises one or a plurality of components selected from the group consisting of *Rubus buergeri, Aster yomena, Stauntonia hexaphylla, Rubus grayana, Kalimeris indica, Lithocarpus edulis, Myrica rubra, Syzygium jambos, Quercus serrata* and extracts of these plants, as well as saffron extract, pine extract, cinchona extract, comfrey extract, Scutellaria extract, rosemary extract, apricot seed powder, gentian extract, thyme extract, mint powder, jujuba fruit extract, hop extract, kiwifruit extract, roman chamomile extract, apple extract, hawthorn extract and turmeric extract.

Moreover, the inventors of the present invention surprisingly found that in a hot flash model of menopausal disorders, apelin demonstrates the ability to contribute to the suppression, improvement, prevention or treatment of hot flashes as was previously described. Thus, an apelin receptor agonist that activates apelin receptors obtained according to the aforementioned screening method is useful for the suppression, improvement, prevention or treatment of hot flashes. Thus, a hot flash suppressant is provided that comprises one or a plurality of components selected from the group consisting of the aforementioned apelin receptor agonists.

*Rubus buergeri* is an evergreen climbing vine shrub that is a type of plant classified as family Rosaceae, genus Poa. It is distributed in on the Japanese islands of Kyushu, Shikoku and Honshu to the west of the Kanto region, as well as in south central China, Taiwan and in the southern region of the Korean peninsula, its fruit ripens from around November to January, and is considered to be edible in the form of a so-called raspberry.

*Aster yomena* is a perennial of the *Asteraceae* family that is distributed in on the Japanese islands of Honshu to the west of the Chubu region, Shikoku and Kyushu. It is typically known to be a type of wild chrysanthemum that produces light violet or white chrysanthemum flowers in the fall. Its young buds are considered to be edible.

*Stauntonia hexaphylla* is an evergreen climbing woody plant belonging to the family Lardizabalaceae, genus Stauntonia, and is also referred to as Japanese staunton vine. It is distributed on the Japanese islands of Honshu to the west of the Kanto region and in Taiwan and China. Its fruit is considered to be edible, and its stem and root have diuretic action and are used as herbal medicines.

*Rubus grayana* is a type of plant classified as family Rosaceae, genus *Rubus* that is a deciduous shrub. It is also referred to as Hainan Elaeocarpus, and is distributed in mountainous regions along the coasts of the Ryukyu Islands to the south of Tanegashima Island and Yakushima Island, and in the moderate and subtropical regions of Taiwan. It flowers from April to May. It has orange fruit that are considered edible.

*Kalimeris indica* is a type of plant classified as family *Asteraceae,* genus *Kalimeris* that is an evergreen perennial. It is native to southern Asia, and is distributed in Japan in Okinawa south of the southern regions of Shikoku and Kyushu. It has a height of about 30 cm and blooms in the fall. Since it is somewhat smaller than *Aster yomena,* it is also referred to as miniature *Aster yomena.* Its young buds are considered to be edible in the same manner as *Aster yomena.*

*Lithocarpus edulis* is an evergreen tree of the Fagaceae family, and is distributed in the southern tip of the Boso peninsula and on the Kii peninsula of the Japanese island of Honshu, and along temperate coastline regions from Kyushu to the Nansei Islands. Since its fruit does not contain much tannin, it is not required to be purged of bitter taste and can be eaten by roasting as is. It is also consumed by grinding into a powder and mixing with cookie dough.

*Myrica rubra* is an evergreen tree of the order *Myricales,* family *Myricaceae* that is native to mainland China and Japan, and its fruit can be consumed raw or is used in foods and beverages such as jam or wine. In addition, it has also been reported to be able to be used as a fat accumulation inhibitor.

*Syzygium jambos* is an evergreen tree of the family Myrtaceae that is native to Southeast Asia, and its fruit, although having little taste, can be eaten because of its rose-like aroma. In addition, it has also been reported to be able to be used as anti-obesity agent.

*Quercus serrata* is a deciduous broad-leaf tree of the order Fagales, family Fagaceae, genus Quercus that is distributed in Hokkaido, Honshu, Shikoku, Kyushu, the Korean peninsula and China, its wood is used as a raw material of charcoal and pulpwood of Shiitake mushrooms, and its fruit are considered to be edible by removing the bitter taste. The fruit has also been reported to be able to be used as an obesity preventive.

Since these nine types of plants are widely distributed and cultivated in Japan as well, they can be acquired easily.

Saffron extract is an extract derived from saffron (*Crocus sativus L.)* that is perennial of the Iridaceae family that is native to the Mediterranean coast and Asia Minor, and the bulbs, roots, leaves, stems, flowers, styles, whole plant, and preferably the styles, can be used as raw materials. Saffron extract contains carotenoid glycosides such as crocin, picrocrocin and crocetin, and is known to have circulation promoting action and skin cell activating action.

Pine extract is an extract derived from evergreen, coniferous trees of the genus *Pinus* such as *Pinus thunbergii* or *Pinus densiflora* that are widely distributed in Siberia, Europe and Asia and are found as common vegetation in Japan as well, and the roots, leaves, bark, strobilus, whole plant, and preferably the bark, of this plant can be used as raw materials. Pine extract contains components such as polysaccharides and amino acids, and is known to have moisturizing action and softening action.

Cinchona extract is an extract derived from evergreen plants of the genus *Cinchona* that are native to tropical regions, and the roots, leaves, bark, flowers, whole plant, and preferably the bark, of this plant can be used as raw materials. Cinchona extract contains components such as quinidine, quinine and cinchonine, and has moisturizing action and circulation promoting action.

Comfrey extract is an extract derived from *Symphtum officinale* that is a perennial plant native to Europe and widely cultivated in Japan as well, and its roots, leaves, stem, flowers, whole plant, and preferably the leaves, can be used as raw materials. Comfrey extract contains components such as allantoin, tannin and vitamins, and is known to have antiphlogistic action and moisturizing action.

Scutellaria extract is an extract derived from the root (scutellaria root) of *Scutellaria baicalensis* that is a perennial grass of the family Lamiaceae, genus *Scutellaria* native to northern mainland China, and is known to have anti-aging action and is used as an active ingredient of therapeutic drugs and cosmetics for lifestyle-related diseases and the like.

Rosemary extract is an aromatic extract derived from *Rosmarinus officinalis* that is an evergreen shrub of the family Lamiaceae native to the Mediterranean coast, and its roots, leaves, stem, flowers and whole plant can be used as raw materials. It is known to have sympathetic nerve activating action and slimming action, and is used as an active ingredient in pharmaceuticals, foods and cosmetics.

Apricot seed powder is a powdered raw material produced by crushing the seeds and endocarp of Prunus armeniaca L., or related plants, which is native to northern mainland China. Apricot seed powder may be used as a dry powder by crushing directly or after drying, or may be used as an extract by extracting these raw materials with a solvent. Apricot seed powder or an extract thereof is known to have fat accumulation inhibitory action and thinning action, and is used as an active ingredient in pharmaceuticals and cosmetics.

Gentian extract is an extract derived from Gentiana lutea, a perennial grass of the family Gentianaceae that is native to Europe, and the roots, leaves, stem, flowers and whole plant of this plant can be used as raw materials. It is known to have fat accumulation inhibitory action and thinning action, and is used as an active ingredient of pharmaceuticals and cosmetics.

Thyme extract is an extract derived from a plant belonging to the family Lamiaceae, genus Thymus, and particularly from common thyme, that is native to Europe, northern Africa and Asia, and its roots, leaves, stem, flowers and whole plant can be used as raw materials. It is known to have fat accumulation inhibitory action, and is used as an active ingredient of pharmaceuticals, foods

and cosmetics.

Mint powder is a powder obtained by drying the entire plant of a perennial of the family Lamiaceae, genus Mentha that is native to the Mediterranean coast, Europe and eastern Asia. It is known to have fat-burning action, and is widely used as an active ingredient of pharmaceuticals, foods and cosmetics.

Jujuba fruit extract is an extract derived from plant of the family Rhamnaceae, genus Ziziphus (Ziziphus jujuba) that is native to Europe, and the whole plant, and particularly the unripened fruit, are used as raw materials. It is known to have thinning action, and is used as an active ingredient of pharmaceuticals, foods and cosmetics.

Hop extract is an extract derived from Humulus lupulus, a plant of the family Moraceae that is native to Europe, and the whole plant, and particularly the spikes, are used as raw materials. It is known to have action that suppresses adipocyte differentiation and antiphlogistic action, and is used as an active ingredient of therapeutic drugs for the treatment of obesity and its accompanying depression and pathological states relating to inflammatory response, as well as in foods and cosmetics.

Kiwifruit extract is an extract derived from Actinidia deliciosa, a dioecious plant of the family Actinidiaceae, genus Actinidia that is native to China, and the whole plant, and particularly its fruit, are used as raw materials. It has been reported to have anti-obesity action, and is used as an active ingredient of therapeutic drugs for the treatment of hypertension, hyperlipemia and pathological obesity, as well as in foods and cosmetics.

Roman chamomile extract is extract derived from Chamaemelum nobile, a plant of the family Asteraceae, genus Anthemis that is native to Europe, and the whole plant, and particularly the flowers, are used as raw materials. It is known to have action that promotes production of dehydroepiandrosterone (DHEA), and has been reported to be able to be used as a pharmaceutical such as skin ameliorants and other anti-aging agents, immunoactivators, anti-diabetic agents, anti-osteoporosis agents, anti-obesity agents, sleep-inducing agents and anti-central nervous system agents.

Apple extract is an extract derived from Malus domestica, a plant of the family Rosaceae, genus Malus that is native to central Asia, and the whole plant, and particularly the fruit, are used as raw materials. It contains procyanidin as an active ingredient, and is reported to be able to be used as an anti-obesity agent.

Hawthorn extract is an extract derived from Crataegus cuneata, a plant of the family Rosaceae, genus Crataegus, and its flowers, fruit and whole plant can be used as raw materials. It is known to have fat-burning promoting action, and is used as an ingredient of foods and beverages.

Tumeric extract is an extract derived from Curcuma longa, a plant of the order Zingiberales, family Curcuma that is known to have fat metabolism ameliorative action and fat accumulation inhibitory action, and is widely used as an ingredient of pharmaceuticals, foods and cosmetics.

Although there are no particular limitations on the method used to extract these plant extracts, an extraction method that uses a solvent is preferable. When carrying out extraction, although the aforementioned raw materials can be used in their original form, crushing or shredding into the form of a powder followed by extracting enables extraction of the active ingredients to be carried out under mild conditions, in a short period of time, and with high extraction efficiency.

Furthermore, the plant body or extract of each of the plants used in the present invention refers to that obtained in the form of a dry powder by crushing as is or after drying, or using as is or drying and crushing followed by extracting with a solvent, the various sites of each plant body (such as the flowers, spikes, skin, fruit, stems, leaves, branches, chutes, trunk, bark, rhizomes, root bark, roots, seeds or the whole plant).

Although any site of the aforementioned plants may be used as extraction sites of the aforementioned plants, preferably, the leaves are used in the case of *Rubus buergeri,* the leaves and stems are used in the case of *Aster yomena,* the stems and roots are used in the case of *Stauntonia hexaphylla,* the leaves are used in the case of *Rubus grayana,* the leaves and stems are used in the case of *Kalimeris indica,* the fruit are used in the case of *Lithocarpus edulis,* the bark, sap, fruit, seeds, flowers, branches, laves or roots, and particularly the leaves and branches, are used in the case of *Myrica rubra,* the bark, sap, fruit, seeds, flowers, branches, leaves and roots, and particularly the leaves and branches, are used in the case of *Syzygium jambos,* and the bark, sap, fruit, seeds, flowers, branches, leaves and roots, and particularly the leaves, bark and roots, are used in the case of *Quercus serrata.*

There are no particular limitations on the extraction temperature, and may be suitably set according to the size of crushed pieces of the raw materials or type of solvent and the like. Normally, the extraction temperature is set within a range from room temperature to the boiling point of the solvent. In addition, there are also no particular limitations on extraction time, and may be suitably set according to the size of the crushed pieces of the raw materials, type of solvent or extraction temperature and the like. Moreover, stirring may be carried out during extraction, the extract may be allowed to stand undisturbed without stirring, or the extract may be subjected to ultrasonic waves.

For example, the aforementioned plant extracts can be obtained by immersing a raw material in a solvent and extracting at room temperature or a temperature of 80°C to 100°C. After filtering the extract obtained by this extraction treatment, it can be used as an active ingredient either directly or after concentrating or drying to a solid as necessary. Furthermore, during this extraction treatment, the raw material may be used after shredding or crushing. In addition, an unprocessed raw material or dried raw material may be used, or a raw material may be used after roasting. There are no particular limitations on the roasting method, and for example, a method be used consisting of roasting for 5 hours to 2 hours at 80°C to 120°C.

There are no particular limitations on the type of solvent used for extraction, and water (including hot water), alcohols (such as methanol, ethanol, 1-propanol, 2-propanol, 1-butanol or 2-butanol), glycols (such as 1,3-butylene glycol or propylene glycol), glycerin, ketones (such as acetone or methyl ethyl ketone), ethers (such as diethyl ether, dioxane, tetrahydrofuran or propyl ether), acetonitrile, esters (such as ethyl acetate or butyl acetate), aliphatic hydrocarbons (such as hexane, heptane or liquid paraffin), aromatic hydrocarbons (such as toluene or xylene) and halogenated hydrocarbons (such as chloroform), either alone or as a mixed solvent of two or types thereof, are preferable.

As a result of carrying out this type of extraction procedure, an active ingredient is extracted from the raw material and dissolved in a solvent. Although a solvent containing the extract may be used as is, it may also be used after aging by allowing to stand undisturbed for several days. Moreover, it may also be used after subjecting to commonly used purification treatments such as sterilization, washing, filtration, decolorization or deodorization. In addition, it may also be used after concentrating or diluting as necessary. Moreover, it may also be used after evaporating the solvent to form a solid (dried product), or may be used after re-dissolving the dried product in an arbitrary solvent.

Moreover, in the case of using the hot flash suppressant or apelin receptor agonist of the present invention as an external skin preparation, commonly used assistants can be suitably incorporated in the external skin preparation, examples of which include metal chelating agents such as disodium edetate, trisodium edetate, sodium citrate, sodium polyphosphate, sodium metaphosphate or gluconic acid, pharmaceutical agents such as caffeine, tannin, verapamil, tranexamic acid and derivatives thereof, licorice extract, licorice root extract, glabridin, hot water extract of Chinese quince fruit, various types of herbal medicines, tocopherol acetate, glycyrrhizinic acid and derivatives thereof or salts thereof, whitening agents such as vitamin C, magnesium ascorbyl phosphate, ascorbic acid glucosides, arbutin or kojic acid, sugars such as glucose, fructose, mannose, saccharose or trehalose, and vitamins such as retinoic acid, retinol, retinol acetate or retinol palmitate.

The hot flash suppressant or apelin receptor agonist according to the present invention is also used in an esthetic method for the purpose of improving hot flashes in persons suffering from hot flashes. This esthetic method can be carried out by, for example, applying the hot flash suppressant according to the present invention to a site presenting with hot flashes. Although examples of the site where hot flashes are presented in this method include all sites throughout the body, the face is particularly preferable.

In another mode of the present invention, the present invention relates to a hot flash suppressant containing a vector that contains incorporating an apelin gene. This suppressant may be such that the gene that encodes apelin (apelin gene) in a subject is in an inactive state or dormant state, and in the case cells are in an apelin-deficient state as a result thereof, is used to introduce the apelin gene per se into cells. In the vector, a regulating sequence that enhances expression of apelin gene, such as a promoter or enhancer, is preferably arranged at a location that is able to act on the apelin gene.

Either a method consisting of gene insertion using a virus vector or a method consisting non-viral gene insertion (Nikkei Science, April 1994, pp. 20-45; Experimental Medicine Special Edition, 12(15) (1994); and, Experimental Medicine Supplement, "Basic Techniques of Gene Therapy", Yodosha Co., Ltd.) can be applied for inserting the apelin gene into cells. Examples of insertion methods using a virus vector include methods consisting of incorporating DNA encoding apelin into a DNA virus or RNA virus such as retrovirus, adenovirus, adeno-associated virus, herpes virus, vaccinia virus, pox virus, polio virus or sindbis virus. Among these, methods using retrovirus, adenovirus, adeno-associated virus or vaccinia virus are particularly preferable. Examples of non-viral insertion methods include a method consisting of directly administering an expression plasmid intramuscularly (DNA vaccination), liposome method, lipofection, microinjection, calcium phosphate method and electroporation, and DNA vaccination and electroporation are particularly preferable. In addition, in order to allow the aforementioned gene to actually as a pharmaceutical, an in vivo method, in which DNA is introduced directly into the body, or an ex vivo method, in which a certain type of cells are removed from a human, DNA is inserted into the cells outside the body, and the cells are then returned to the body (Nikkei Science, April 1994, pp. 20-45; Experimental Medicine Special Edition, 12(15) (1994); and, Pharmaceuticals Monthly, 36(1), 23-48 (1994)). However, an in vivo method is preferable. In the case of administering by an in vivo method, administration can be carried out by a suitable administration route corresponding to the disease, symptoms and the like. For example, administration can be carried out intravenously, intraarterially, subcutaneously or intramuscularly. In the case of administering by an in vivo method, an injection preparation is typically used, and a commonly used vehicle may be added as necessary. In addition, in the case of administering in the form of liposomes or membrane-fused liposomes (such as Sendai virus (HVJ)-liposomes), administration can be carried out in the form of a liposome preparation such as a suspension, frozen preparation or centrifuged, concentrated frozen preparation.

Thus, in another embodiment of the present invention, a suppressant is provided for suppressing or improving hot flashes that comprises a vector containing a gene encoding apelin or an apelin receptor agonist. In this case, the gene encoding apelin is preferably coupled to a regulating sequence that enhances expression of apelin gene so that it is able to act thereon.

Although the incorporated amount of vector comprising a gene encoding apelin of the present invention (suppressant of the present invention) in a preparation can be suitably determined according to the application, it is typically 0.00001% by weight to 20.0% by weight, and preferably 0.00001% by weight to 10.0% by weight in the preparation.

The following provides a more detailed explanation of the present invention through examples thereof. Furthermore, the present invention is not limited thereto.

### Examples

### Example 1: Hot Flash Suppressive Action of Apelin

Ovariectomy was performed on C57/BL6 mice highly expressing apelin in the epidermis under the control of a K14 promoter (herein after referred to as apelin-highly expressed mice, apelin TG or TG) and the corresponding wild-type mice (C57/BL6) (n=5, 8 to 10 week-old) to obtain menopausal mice highly expressing apelin and menopausal wild type mice. Furthermore, wild type mice that underwent sham surgery were used as a control. Four weeks later, nifedipine (50 µg/kg) was administered intraperitoneally as a vasodilator, and changes in tail temperature were analyzed using thermography (Thermo Tracer TH9011MR, NEC/Avio Infrared Technologies Co., Ltd.) at 10, 30 and 45 minutes after administration.

### Results

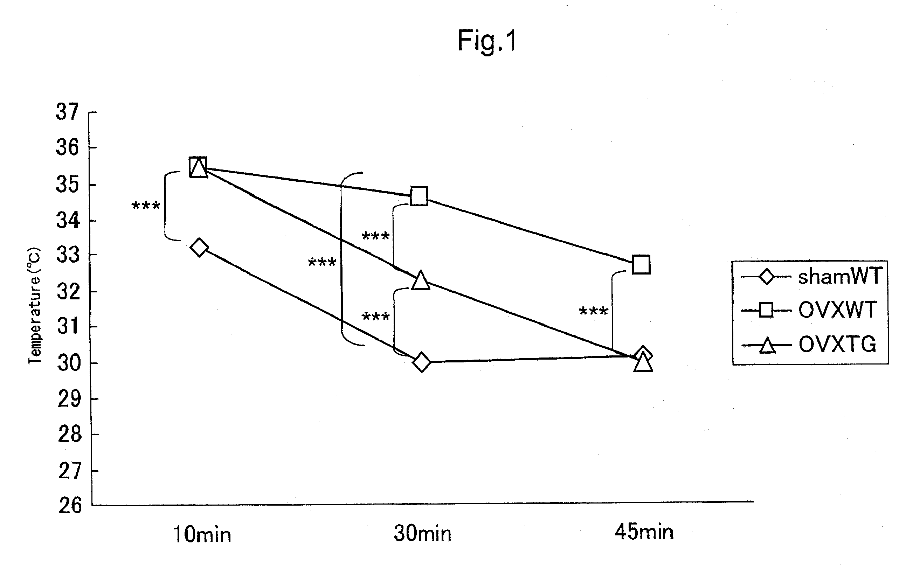

In the control wild type mice (shamWT), skin temperature that rose 10 minutes after administration of vasodilator returned to normal 30 minutes later and remained constant thereinafter (FIG. 1). On the other hand, in the menopausal wild type mice (OVXWT) and menopausal mice highly expressing apelin (OVXTG), body temperature rose significantly after 10 minutes after administration of vasodilator in comparison with the control (shamWT), and in the OVXWT mice, body temperature was significantly higher in comparison with both the shamWT and OVXTG mice at 30 minutes and 45 minutes after administration. On the other hand, in the OVXTG mice, body temperature decreased in comparison with the OVXWT mice after 30 minutes, and body temperature decreased to roughly the same temperature as that of the control shamWT mice after 45 minutes. A model of menopausal hot flashes was able to be developed by of measuring the tail temperatures of menopausal mice administered vasodilator (Kai, M., Yamauchi, A., Tominaga, K., Koga, A., Kai, H., Kataoka, Y.: Soybean isoflavones eliminate nifedipine-induced flushing of tail skin in ovariectomized mice, J. Pharmacol. Sci. 2004, 95: 476-478). Thus, the above results suggested that apelin is able to suppress or improve transient rises in body temperature caused by hot flashes in a short period of time.

### Example 2: Establishment of Screening System for Apelin-like Pharmaceutical Agents

### Culturing of APJ-Expressing Cells

A cAMP luciferase reporter vector was transfected into a stable cell line, which was obtained by highly expressing Apelin receptor APJ in NIH3T3 cells, and then subjecting to selection with hygromycin (provided by Professor Nobuyuki Takakura of the Research Institute for Microbial Diseases of Osaka University), and these were used in the cAMP luciferase assay described below. Culturing was carried out in DMEM medium containing 10% FBS containing 200 µg/ml of hygromycin (Invitrogen). On the day before the assay, 2.0 x 10⁵ cells were seeded into each well of a 96-well plate followed by culturing in DMEM medium containing 10% FBS and 200 µg/ml of hygromycin.

### cAMP Luciferase Assay

After culturing overnight, the medium was removed by aspiration and replaced with 100 µl of additive-free DMEM per well. Three hours later, Glosensor™ cAMP Reagent (Promega) was added at 4 µl per well followed by equilibrating for 2 hours at room temperature under light blocking. Subsequently, apelin 13 (Peptide Institute) was diluted with additive-free DMEM medium to a concentration of 0.4 µg/ml and this was added at 50 µl per well (final concentration: 0.1 µg/ml) followed by incubating for 5 minutes at room temperature. Moreover, 50 µl aliquots of forskolin adjusted to a concentration of 40 µM (Sigma) were added (final concentration: 10 µM) followed by treating for 15 minutes. Subsequently, luminescence was measured with the GloMax™ 96 Microplate Luminometer (Promega). In addition, a luciferase assay was carried out in the same manner as described above by adding apelin neutralizing antibody 4G5 (provided by Osaka University) together with apelin. In this case, the apelin was diluted with DMEM medium not containing neutralizing antibody to a concentration of 5 µg/ml, and 50 µl aliquots were added to each well (final concentration: 0.5 µg/ml). Similarly, neutralizing antibody was prepared to final concentrations of 1.5, 5, 15 and 50 µg/ml and added to their respective wells. Forskolin having a concentration of 20 µg/ml was added at 100 µl per well without adding apelin was used as a control.

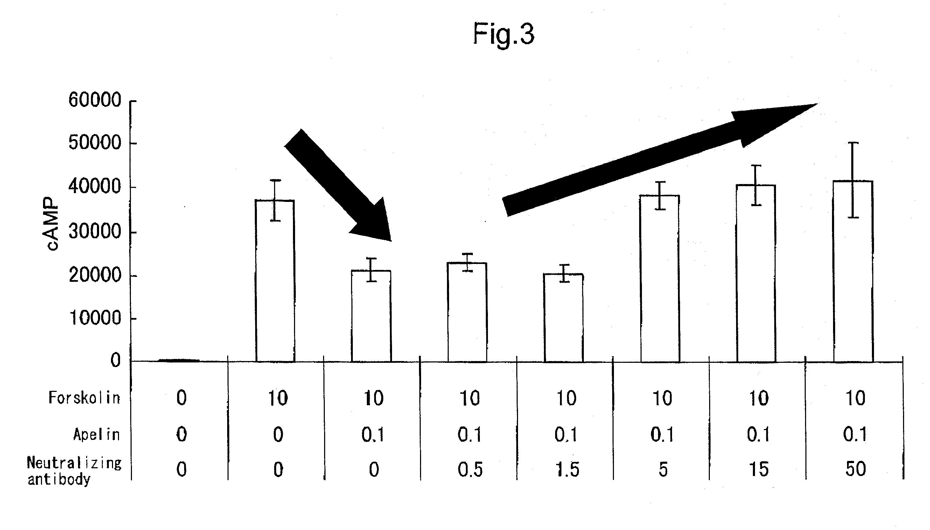

The results of the aforementioned cAMP luciferase assay are shown in FIG. 3. As can be understood from FIG. 3, although cAMP concentration is decreased by apelin, decreases in cAMP concentration are suppressed accompanying loss of apelin activity caused by the apelin neutralizing antibody. Namely, in the aforementioned cAMP luciferase assay, a search is first made of a pharmaceutical agent that decreases cAMP concentration in the case of having added candidate pharmaceutical agents instead of apelin (primary screening), and as a result of next further screening for a pharmaceutical agent among these pharmaceutical agents that significantly suppresses the decrease in cAMP concentration in the case of having added together with apelin neutralizing antibody (secondary screening), a pharmaceutical agent that decreases cAMP concentration through activation of apelin receptor in the form of APJ (namely, an apelin-like pharmaceutical agent) can be selected efficiently and in a short period of time.

### Example 3: Screening of Apelin-like Pharmaceutical Agents (1)

Apelin-like pharmaceutical agents were selected according to the screening system established in Example 2 using about 250 types of cosmetic materials (Shiseido) as candidate pharmaceutical agents.

More specifically, each candidate pharmaceutical agent was prepared to a concentration of 400 µg/ml, and an assay was carried out in the same manner as Example 2 by adding 50 µl aliquots of the candidate pharmaceutical agents (final concentration: 100 µg/ml) to each well instead of the apelin used in the cAMP luciferase assay of Example 2.

As shown in FIG. 4, saffron extract, pine extract, cinchona extract, comfrey extract, Scutellaria extract, rosemary extract, apricot seed powder, gentian extract, thyme extract, mint powder, jujuba fruit extract, hop extract, kiwifruit extract, roman chamomile extract, apple extract, hawthorn extract and turmeric extract significantly reduced intracellular cAMP concentration, and since the reductions in cAMP concentration were significantly suppressed due to loss of apelin activity in the case of having added together with apelin neutralizing antibody (4G5), these plant extracts were demonstrated to have a function similar to that of apelin.

### Example 4: Screening of Apelin-like Pharmaceutical Agents (2)

Apelin-like pharmaceutical agents were selected according to the screening system established in Example 2 using about 110 types of food materials (Shiseido) as candidate pharmaceutical agents.

More specifically, each candidate pharmaceutical agent was prepared to a concentration of 400 µg/ml, and an assay was carried out in the same manner as Example 2 by adding 50 µl aliquots of the candidate pharmaceutical agents (final concentration: 100 µg/ml) to each well instead of the apelin used in the cAMP luciferase assay of Example 2.

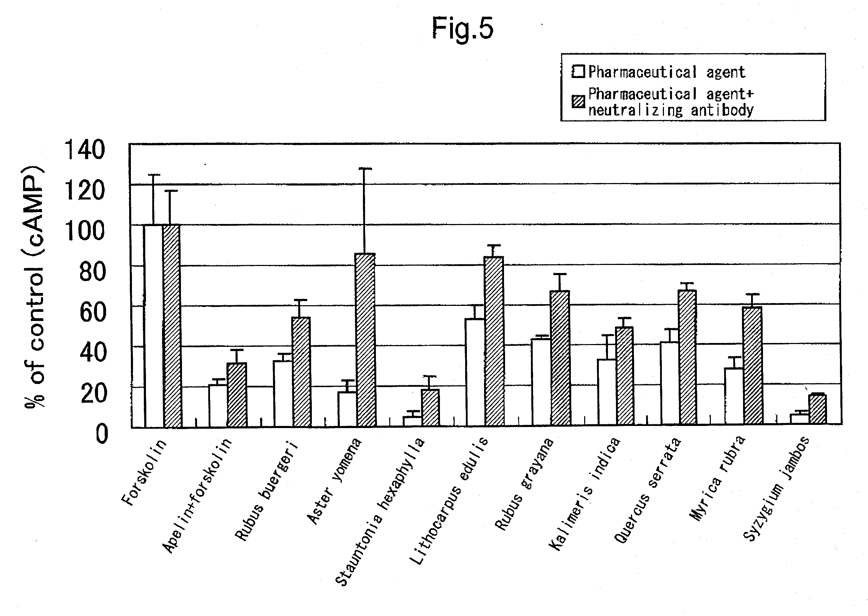

As shown in FIG. 5, extracts of *Rubus buergeri, Aster yomena, Stauntonia hexaphylla, Rubus grayana, Kalimeris indica, Lithocarpus edulis, Myrica rubra, Syzygium jambos, Quercus serrata* significantly reduced intracellular cAMP concentration, and since the reductions in cAMP concentration were significantly suppressed due to loss of apelin activity in the case of having added together with apelin neutralizing antibody (4G5), these plant extracts were demonstrated to have a function similar to that of apelin.

## Claims

1. A hot flash suppressant comprising apelin or an apelin receptor agonist.

2. The hot flash suppressant according to claim 1, wherein the apelin is:
(a) a protein composed of the amino acid sequence represented by SEQ ID NO: 1, or
(b) a protein composed of an amino acid sequence, in which one or several amino acids in the amino acid sequence of SEQ ID NO: 1 have been deleted, substituted or added, and having an apelin activity.

3. The hot flash suppressant according to claim 1, wherein the apelin receptor agonist is selected from the group consisting of *Rubus buergeri, Aster yomena, Stauntonia hexaphylla, Rubus grayana, Kalimeris indica, Lithocarpus edulis, Myrica rubra, Syzygium jambos, Quercus serrata* and extracts of these plants, as well as saffron extract, pine extract, cinchona extract, comfrey extract, Scutellaria extract, rosemary extract, apricot seed powder, gentian extract, thyme extract, mint powder, jujuba fruit extract, hop extract, kiwifruit extract, roman chamomile extract, apple extract, hawthorn extract and turmeric extract.

4. A method for treating, improving or preventing hot flashes, comprising administration of apelin or an apelin receptor agonist.

5. The method for treating, improving or preventing hot flashes according to claim 4, wherein the apelin is:
(a) a protein composed of the amino acid sequence represented by SEQ ID NO: 1, or
(b) a protein composed of an amino acid sequence, in which one or several amino acids in the amino acid sequence of SEQ ID NO: 1 have been deleted, substituted or added, and having an apelin activity.

6. The method for treating, improving or preventing hot flashes according to claim 4, wherein the apelin receptor agonist is selected from the group consisting of *Rubus buergeri, Aster yomena, Stauntonia hexaphylla, Rubus grayana, Kalimeris indica, Lithocarpus edulis, Myrica rubra, Syzygium jambos, Quercus serrata* and extracts of these plants, as well as saffron extract, pine extract, cinchona extract, comfrey extract, Scutellaria extract, rosemary extract, apricot seed powder, gentian extract, thyme extract, mint powder, jujuba fruit extract, hop extract, kiwifruit extract, roman chamomile extract, apple extract, hawthorn extract and turmeric extract.

7. A method for screening hot flash suppressants by using an apelin receptor activity or apelin expression as an indicator.
